# EUROPEAN PATENT SPECIFICATION

(11) **EP 3 275 503 B1**
(45) Date of publication and mention of the grant of the patent: **22.01.2025**
(21) Application number: 17189436.3
(22) Date of filing: 19.03.2007
(51) Int. Cl.: A61M 37/00, A61M 39/02

(54) **VENOUS ACCESS PORT ASSEMBLY WITH RADIOPAQUE INDICIA**
VENENZUGANGSÖFFNUNGS-ANORDNUNG MIT RÖNTGENOPAKEN MARKIERUNGEN
ENSEMBLE D'ORIFICES D'ACCÈS VEINEUX AVEC DES REPÈRES RADIOPAQUES

(30) Priority: 18.10.2006 US 852591 P
(43) Date of publication of application: 31.01.2018
(62) Divisional of application: 07753408.9
(73) Proprietor: Medical Components, Inc., Harleysville, PA 19438 (US); Innovative Medical Devices, LLC, Westport, CT 06880 (US)
(72) Inventor: ZINN, Kenneth M., Westport, CT 06880 (US); BIZUP, Raymond, Feasterville, PA 19053 (US); SANFORD, Kevin, Chalfont, PA 18914 (US); SCHWEIKERT, Timothy, Levittown, PA 19054 (US)
(74) Representative: Mewburn Ellis LLP

(56) References cited:
- WO-A1-2006/096686
- US-A1- 2004 181 186
- US-B1- 6 287 293

## Description

### FIELD OF THE INVENTION

This relates to the field of medical devices and more particularly to venous access ports for the infusion of fluids into the patient and/or withdrawal of fluids from the patient.

### BACKGROUND OF THE INVENTION

Venous access ports for the infusion and/or withdrawal of fluids from a patient are well-known, secured to the proximal end of an implanted catheter. These ports are typically used for drug infusion or for withdrawal of small amounts of blood, where large flows of fluid are not required. The ports are assemblies of a needle-impenetrable housing with a discharge port in fluid communication with the catheter and the reservoir within the port housing, and provide a subcutaneous self-sealing septum that defines an access site for multiple needle sticks through the covering skin tissue of the patient, through the septum and into the reservoir, without the need to continuously search for new access sites. Examples of such ports are disclosed, for example, in U.S. Patents Nos. 4,704,103; 4,762,517; 4,778,452; 5,185,003; 5,213,574 and 5,637,102.

It is desired to provide a venous access port assembly that provides for a radiologist, radiology technologist, nurse and ultimately a medical practitioner to be able to discern an important property of the port assembly after the port assembly has been implanted into a patient.

US 6 287 293 B1 proposes a method and apparatus for locating the injection point of an implanted medical device.

WO 2006/096686 A1 proposes an access port for subcutaneous implantation and methods of identifying a subcutaneously implanted access port.

US 2004/181186 A1 proposes a stent with rapid return and strong shape memory configured to prevent migration of the stent out of a desired site within a patient's body.

### BRIEF SUMMARY OF THE INVENTION

The invention is as defined in independent claim 1. Preferred embodiments of the invention are as defined in dependent claims 2-10.

The present invention is related to a venous access port having a housing and a septum, providing an interior reservoir and a passageway extending from the reservoir through a stem of a discharge port to establish fluid communication with a proximal end of a catheter lumen to which the port assembly is secured prior to placement of the assembly into a patient. The port may optionally have more than one reservoir and associated septum. The invention is the application of radiopaque indicia onto a venous access port that is discernible under X-ray examination to provide information concerning the nature or key attribute of the venous access port, so that the practitioner, subsequent to the date of implantation thereof, can determine that nature or key attribute under X-ray examination. One such key attribute in particular is that the venous access port is rated to be used for power injection such as of contrast fluid, wherein the letters "CT" (for "computed tomography", or "contrast enhanced computed tomography") are provided that are of radiopaque material, optionally positioned within radiopaque circles. The attribute in this example is the property of the port's being adapted to withstand high pressures that are used for injection of contrast fluid into a patient, and the letters "CT" would be understood in medical practice to indicate that the port is suitable for the high pressure injection of contrast fluid. The radiopaque indicia could for example be applied in a mirror-image orientation on the bottom housing surface, and would appear on the X-ray as right-side up and easily readable by the radiologist, technologist or practitioner.

### BRIEF DESCRIPTION OF THE DRAWINGS

The accompanying drawings, which are incorporated herein and constitute part of this specification, illustrate the presently preferred embodiments of the invention, and, together with the general description given above and the detailed description given below, serve to explain the features of the invention. In the drawings:
Fig. 1 is an isometric view of the venous access port of the present invention;
Fig. 2 is a plan view of the port of Fig. 1;
Figs. 3 and 4 are cross-section views of the port of Figs. 1 and 2 taken along lines 3 - 3 and lines 4 - 4 of Fig. 1, respectively;
Fig. 5 is an isometric view of the needle-impenetrable housing base of the venous access port of Fig. 1;
Figs. 6 and 7 are transverse cross-sectional and longitudinal cross-sectional views of the housing base of Fig. 5;
Fig. 8 is an isometric view from below of the housing base of Figs. 6 and 7, showing the radiopaque indicia applied on the housing base bottom surface; and
Figs. 9 and 10 are bottom and top views of the housing base of Fig. 8 having radiopaque indicia thereon, with the top view being analogous to the X-ray view of the venous access port by the radiologist, and the indicia being shown in dashed lines in Fig. 10.

### DETAILED DESCRIPTION OF THE INVENTION

Certain terminology is used herein for convenience only and is not to be taken as a limitation on the present invention. The terms "distal" and "proximal" refer, respectively, to directions closer to and away from the insertion tip of a catheter in an implantable catheter assembly. The terminology includes the words specifically mentioned, derivatives thereof and words of similar import. The embodiments illustrated below are not intended to be exhaustive or to limit the invention to the precise form disclosed. These embodiments are chosen and described to best explain the principle of the invention and its application and practical use and to enable others skilled in the art to best utilize the invention.

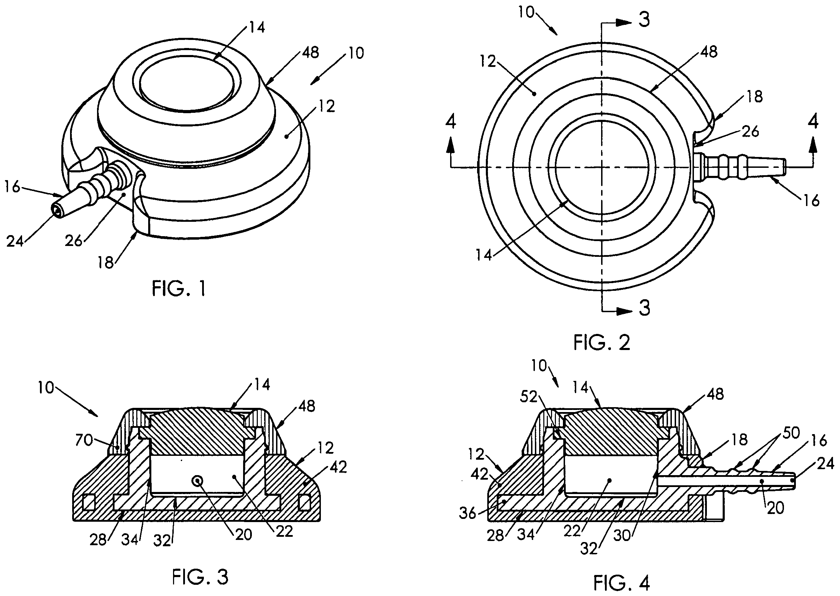

Venous access port assembly 10 of Figs. 1 to 4 includes a housing 12 and a septum 14, with a discharge port 16 extending from a distal end 18 of the port assembly 10 to be attached securely and sealingly to the proximal end of a catheter (not shown). A passageway 20 extends from the interior reservoir 22 to the distal tip opening 24 of discharge port 16. A recess 26 is seen to be provided along both sides of discharge port 16, facilitating insertion of the discharge port 16 into the catheter lumen and providing a clearance for a locking sleeve or clamp (not shown) utilized to compress the catheter lumen wall against the exterior surface of the discharge port 16 for assured sealed connection of the catheter with the port assembly 10.

With reference now to Figures 3 to 7, the interior of the port assembly 10 is shown to provide an interior reservoir 22. Housing 12 is shown to include a housing base 28 of needle-impenetrable material that includes a well 30 having a bottom floor 32 and side walls 34 that define the interior reservoir 22 beneath septum 14. Bottom floor 32 may be convex or elevated (not shown) toward the center of the reservoir, if desired. Housing base 28 includes a base flange 36 extending radially outwardly from the bottom of well 30, and base flange 36 includes openings 38,40 that serve to enable suturing to the patient upon placement of the venous access port and the attached catheter into the patient.

As shown in Figures 3 and 4, a skirt 42 is overmolded about housing base 28 and may be of silicone elastomer. It is seen that skirt 42 encapsulates the outer surfaces of the bottom wall 44 and the bottom portion of the side walls 46 of housing base 28, and is shown to fill in the suture holes 38, 40; but since the material is silicone elastomer, suturing is possible since the suturing needle can easily be inserted through the material of skirt 42 and through the suture holes, and thereafter the filled openings provide minimal opportunity for ingrowth of patient tissue into the openings.

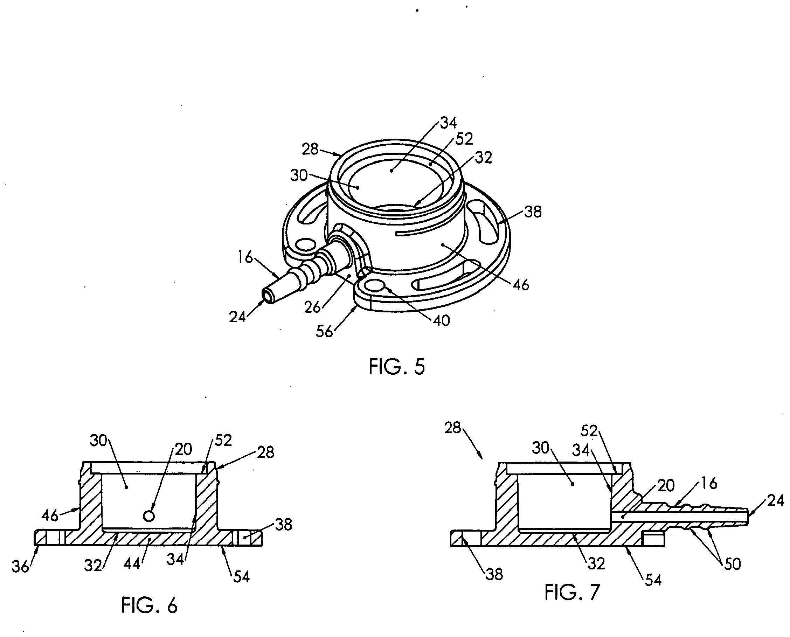

Also seen in Figures 1 to 4 is cap 48, which secures to housing base 28 to in turn secure septum 14 in position in the port assembly 10. Preferably, skirt 42 is insert molded onto base flange 36 of housing base 28 after cap 48 is secured to the upper portion of housing base 28 to secure the septum in position. It is seen in Figures 4 and 7 that discharge port 16 is integral with housing base 28 as is preferable. Discharge port 16 is shown to have a pair of annular ridges 50 that facilitate with the mechanical connection of the catheter proximal end with the port assembly 10. Housing base 28 includes a septum seat 52 extending into the top of well 30, into which a flange of the septum will be seated, preferably under radially inward compression. Housing base 28 has a bottom outer surface 54.

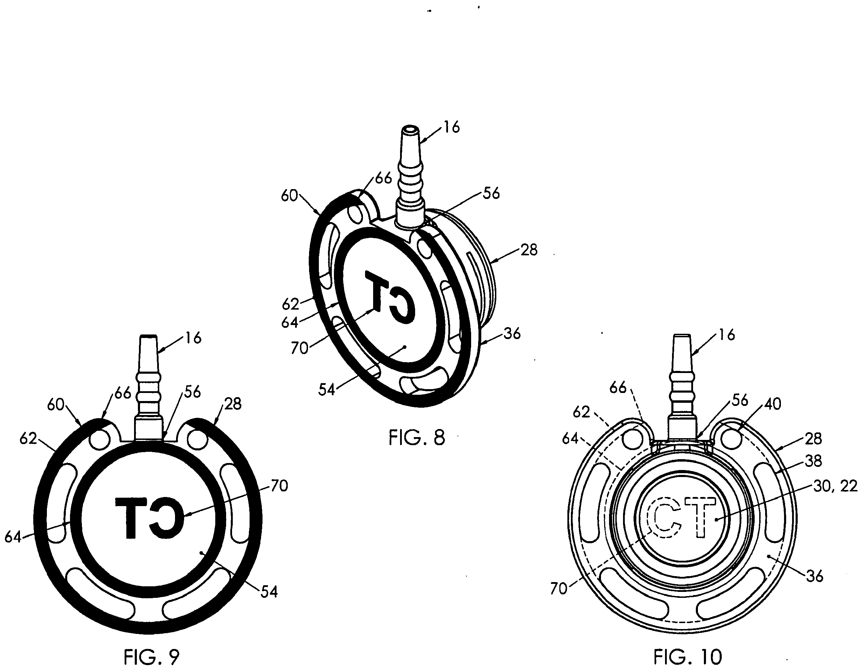

Radiopaque markings 60 of the present invention are shown in Figures 8 to 10. A larger outer circle 62 is seen provided on the outermost periphery of bottom base surface 54, and a smaller inner circle 64 is seen provided within the area circumscribed by the suture openings 38 and holes 40 through base flange 36. Adjacent to discharge port 16, a recess 56 is provided in the skirt of the housing base to provide a clearance for use of a connection sleeve that will be used to secure the catheter (not shown), and outer circle 62 is shown to have a gap 66 at the recess. Outer and inner circles or rings 62, 64 circumscribe radiopaque indicia 70.

Radiopaque indicia 70 are provided on bottom outer surface 54 within the region directly beneath the reservoir and septum. In the example shown, indicia 70 comprise the letters "CT" (Fig. 10) representing the term "computed tomography." The meaning of this particular example of indicia is that the venous access port assembly 10 is rated for high pressure injection such as is necessary for infusion into the patient of contrast medium that is used in computed tomography. Other indicia may of course be used that indicate some other attribute or characteristic of the venous access port assembly. The radiopaque markings and indicia would appear on an X-ray of the patient, and the indicia are provided in a mirror-image orientation, on the bottom outer surface of the housing base (Figs. 7 and 8) so that the indicia would appear as "CT" when the X-ray is viewed (Fig. 9), easily discerned by the radiologist or technologist. Centering of the indicia within the region (identified as "30, 22" in Fig. 10) directly beneath the reservoir and septum minimizes any obscuring by the structure of the venous access port assembly, and the indicia may also be easily discernable should the port assembly be at an angle from the horizontal plane of the X-ray; the outer and inner circles 62, 64 would appear oval or elliptical should the port assembly be at such an angle. Gap 66 in outer circle 62 would also appear and would indicate the location of the discharge port stem 16.

The radiopaque markings may constitute marking fluid that is embossed or imprinted or otherwise applied onto the surface of the housing base 28, such as black radiopaque ink Part No. Cl 1002 Rev A formulated by Creative Imprinting of Erie, Pennsylvania, from Marabu Tampapur TPU 910 clear with tungsten added, available from Marabuwerke GmbH & Co. KG of Stuttgart, Germany, and may be applied on plasma-treated surfaces. At least the housing base 28, the septum 14 and the skirt 42 are of radiotransparent or radiolucent material as is well known in implanted medical devices, and the housing base may be molded of polysulfone resin.

The radiopaque markings may alternatively be applied to the inwardly facing surface of the bottom wall of the housing base these alternatives not being shown in the drawings.

## Claims

1. A venous access port assembly (10), including:
a housing (12) made of needle impenetrable material, the housing (12) further including:
a housing base (28);
a well (30) having a bottom floor (32) and side walls (34) that define an interior reservoir (22); and
a passageway (20) that extends from the interior reservoir (22) to a distal tip opening (24) of a discharge port (16);
wherein radiopaque markings (60, 70) are applied to a surface of the housing base (28); and
**characterized in that** the venous access port assembly (10) is rated for power injection such as of contrast fluid, the radiopaque markings (60, 70) including indicia (70) that comprise the letters "CT" and conveying, when an X-ray of the patient is taken after implantation of the venous access port assembly (10), that the venous access port assembly (10) can be used for power injection.

2. The venous access port assembly (10) recited in claim 1, wherein the radiopaque markings (60, 70) constitute marking fluid.

3. The venous access port assembly (10) recited in any of the preceding claims, wherein the housing base (28) further includes a bottom wall (44) and side walls (46).

4. The venous access port assembly (10) recited in claim 3, wherein the radiopaque markings (60, 70) are applied to an inwardly facing surface of the bottom wall (44).

5. The venous access port assembly (10) recited in any of the preceding claims, wherein the housing base (28) includes a septum seat (52).

6. The venous access port assembly (10) recited in claim 5, wherein the septum seat (52) extends into a top of the well (30).

7. The venous access port assembly (10) recited in any of the preceding claims, wherein the bottom floor (32) is convex or elevated toward a center of the interior reservoir (22).

8. The venous access port assembly (10) recited in any of the preceding claims, wherein the housing base (28) includes a base flange (36).

9. The venous access port assembly (10) recited in claim 8, wherein the base flange (36) extends radially outward from a bottom of the well (30).

10. The venous access port assembly (10) recited in claim 8 or 9, wherein the base flange (36) includes openings (38, 40).

## Patentansprüche

1. Venenzugangsöffnungsanordnung (10), umfassend:
ein Gehäuse (12), das aus einem für Nadeln undurchdringlichen Material besteht, wobei das Gehäuse (12) ferner Folgendes umfasst:
eine Gehäusebasis (28);
eine Vertiefung (30) mit einer Bodenebene (32) und Seitenwänden (34), die einen Innenbehälter (22) definieren; und
einen Durchgang (20), der sich vom Innenbehälter (22) aus zu einer distalen Spitzenöffnung (24) eines Auslassanschlusses (16) erstreckt;
wobei strahlenundurchlässige Markierungen (60, 70) auf einer Oberfläche der Gehäusebasis (28) aufgebracht sind; und
**dadurch gekennzeichnet, dass** die Venenzugangsöffnungsanordnung (10) für eine Leistungsinjektion, wie etwa von Kontrastfluid, ausgelegt ist, wobei die strahlenundurchlässigen Markierungen (60, 70) Zeichen (70) umfassen, welche die Buchstaben "CT" umfassen und, wenn nach dem Implantieren der Venenzugangsöffnungsordnung (10) ein Röntgenbild des Patienten aufgenommen wird, vermitteln, dass die Venenzugangsöffnungsanordnung (10) für die Leistungsinjektion verwendet werden kann.

2. Venenzugangsöffnungsanordnung (10) nach Anspruch 1, wobei die strahlenundurchlässigen Markierungen (60, 70) Markierfluid bilden.

3. Venenzugangsöffnungsanordnung (10) nach einem der vorangegangenen Ansprüche, wobei die Gehäusebasis (28) ferner eine Bodenwand (44) und Seitenwände (46) umfasst.

4. Venenzugangsöffnungsanordnung (10) nach Anspruch 3, wobei die strahlenundurchlässigen Markierungen (60, 70) auf einer nach innen gewandten Oberfläche der Bodenwand (44) aufgebracht sind.

5. Venenzugangsöffnungsanordnung (10) nach einem der vorangegangenen Ansprüche wobei die Gehäusebasis (28) einen Septumsitz (52) umfasst.

6. Venenzugangsöffnungsanordnung (10) nach Anspruch 5, wobei der Septumsitz (52) sich in einen Oberteil der Vertiefung (30) hinein erstreckt.

7. Venenzugangsöffnungsanordnung (10) nach einem der vorangegangenen Ansprüche, wobei die Bodenebene (32) in Richtung eines Mittelpunkts des Innenbehälters (22) konvex oder erhöht ist.

8. Venenzugangsöffnungsanordnung (10) nach einem der vorangegangenen Ansprüche, wobei die Gehäusebasis (28) einen Basisflansch (36) umfasst.

9. Venenzugangsöffnungsanordnung (10) nach Anspruch 8, wobei der Basisflansch (36) sich von einem Boden der Vertiefung (30) aus radial nach außen erstreckt.

10. Venenzugangsöffnungsanordnung (10) nach Anspruch 8 oder 9, wobei der Basisflansch (36) Öffnungen (38, 40) umfasst.

## Revendications

1. Ensemble d'orifice d'accès veineux (10), incluant :
un logement (12) réalisé en matériau ne pouvant être pénétré par une aiguille, le logement (12) incluant en outre :
une base de logement (28) ;
un puits (30) présentant un plancher de fond (32) et des parois latérales (34) qui définissent un réservoir intérieur (22) ; et
un passage (20) qui s'étend depuis le réservoir intérieur (22) vers une ouverture de pointe distale (24) d'un orifice de décharge (16) ;
dans lequel des marques radio-opaques (60, 70) sont appliquées sur une surface de la base de logement (28) ; et
**caractérisé en ce que** l'ensemble d'orifice d'accès veineux (10) est conçu pour une injection de puissance telle qu'un fluide de contraste, les marques radio-opaques (60, 70) incluant des indices (70) qui comprennent les lettres "CT" et véhiculant, lorsqu'une radiographie du patient est prise après l'implantation de l'ensemble d'orifice d'accès veineux (10), que l'ensemble d'orifice d'accès veineux (10) peut être utilisé pour une injection de puissance.

2. Ensemble d'orifice d'accès veineux (10) selon la revendication 1, dans lequel les marques radio-opaques (60, 70) constituent un fluide de marquage.

3. Ensemble d'orifice d'accès veineux (10) selon l'une quelconque des revendications précédentes, dans lequel la base de logement (28) inclut en outre une paroi de fond (44) et des parois latérales (46).

4. Ensemble d'orifice d'accès veineux (10) selon la revendication 3, dans lequel les marques radio-opaques (60, 70) sont appliquées sur une surface tournée vers l'intérieur de la paroi inférieure (44).

5. Ensemble d'orifice d'accès veineux (10) selon l'une quelconque des revendications précédentes, dans lequel la base de logement (28) inclut un siège de septum (52).

6. Ensemble d'orifice d'accès veineux (10) selon la revendication 5, dans lequel le siège de septum (52) s'étend dans une partie supérieure du puits (30).

7. Ensemble d'orifice d'accès veineux (10) selon l'une quelconque des revendications précédentes, dans lequel le plancher de fond (32) est convexe ou surélevé vers un centre du réservoir intérieur (22).

8. Ensemble d'orifice d'accès veineux (10) selon l'une quelconque des revendications précédentes, dans lequel la base de logement (28) inclut une bride de base (36).

9. Ensemble d'orifice d'accès veineux (10) selon la revendication 8, dans lequel la bride de base (36) s'étend radialement vers l'extérieur à partir d'un fond du puits (30).

10. Ensemble d'orifice d'accès veineux (10) selon la revendication 8 ou 9, dans lequel la bride de base (36) inclut des ouvertures (38, 40).
